# EUROPEAN PATENT APPLICATION

(11) **EP 4 733 401 A1**
(43) Date of publication of application: **29.04.2026**
(21) Application number: 24825325.4
(22) Date of filing: 21.06.2024
(51) Int. Cl.: C12N 15/70, C12N 15/113, C12N 15/11

(54) **METHOD FOR PREPARING SINGLE-STRANDED DNA USING CAS NICKING ENZYME**

(30) Priority: 21.06.2023 CN 202310743161
(71) Applicant: Nanjing GenScript Biotech Co., Ltd., Nanjing, Jiangsu 211100 (CN)
(72) Inventor: ZHOU, Fan, Nanjing, Jiangsu 211100 (CN); ZHANG, Lu, Nanjing, Jiangsu 211100 (CN); YE, Lumeng, Nanjing, Jiangsu 211100 (CN); LI, Hong, Nanjing, Jiangsu 211100 (CN)
(74) Representative: Gille Hrabal Partnerschaftsgesellschaft mbB Patentanwälte
(86) International application number: PCT/CN2024/100559
(87) International publication number: WO 2024/260438

(57) **Abstract**

A method for preparing a single-stranded DNA using a CAS nicking enzyme. The method solves the problem of limited enzyme cleavage sites when a traditional nickase is used, and multiple gRNAs can be used in one enzyme digestion system for multi-point nicking. The plasmid nicking efficiency is improved, and the digestion efficiency of the subsequent exonuclease is also effectively improved.

## Description

### CROSS-REFERENCE TO RELATED APPLICATIONS

The present invention claims priority to the prior application with the patent application number 2023107431619 filed with the China National Intellectual Property Administration on June 21, 2023 and entitled "METHOD FOR PREPARING SINGLE-STRANDED DNA USING CAS NICKING ENZYME", which is incorporated herein by reference in its entirety.

### TECHNICAL FIELD

The present invention relates to the field of biochemistry, and in particular relates to a preparation method for a single-stranded DNA, particularly a circular single-stranded DNA, and a related product and kit.

### BACKGROUND

The CRISPR/Cas system plays an extremely important role in the field of modern bioscience, and its emergence has revolutionized genome editing technology. Gene knock-in based on the homology directed repair (HDR) pathway is one of the important applications of the CRISPR/Cas system. The template-dependent manner means that the suitability of the HDR template determines the success and efficiency of gene knock-in. The commonly used double-stranded DNA (dsDNA) templates have the problems of low efficiency, high off-target rate and significant cytotoxicity. Single-stranded DNA (ssDNA) has been proven to be an ideal knock-in template in HDR-based gene knock-in experiments. As a knock-in template, ssDNA exhibits advantages such as superior editing efficiency, lower cytotoxicity, and reduced off-target effects. Compared to linear single-stranded DNA (lssDNA), circular single-stranded DNA (cssDNA) exhibits higher stability, enables effective and stable insertion of a fragment of interest, and has a high targeting rate. Additionally, a cssDNA donor outperforms a lssDNA donor in template-driven repair at a target site.

Currently, the synthesis methods for ssDNA can be classified into chemical synthesis, bacterial-based synthesis, and enzymatic synthesis (see: doi: 10.3390/genes11020116; US 10940171 B2). Chemical synthesis has limitations on the length of ssDNA, incurs high costs, and requires additional purification methods. Phage-based synthesis enables large-scale production of ssDNA with arbitrary sequences in bioreactors, but this method is time-consuming and not suitable for rapid prototyping (see: US 20200362332). Enzymatic synthesis is a relatively low-cost and rapid synthesis method for ssDNA. This method has less limitations on the length of the synthesized sequence, enabling efficient synthesis of longer fragments.

For traditional enzymatic synthesis method for cssDNA, one approach involves first obtaining lssDNA, followed by cyclization using a ligase to obtain cssDNA (see: EP 2610352 B1; and CN 107002292 A). Another approach involves nicking the non-target strand of a double-stranded plasmid with a nickase (e.g., Nb.BtsI, Nt.BspQI, or Nb.Bpu10I), followed by digesting the nicked DNA strand with an exonuclease to ultimately obtain the target cssDNA (Zhang et al., Engineering BspQI nicking enzymes and application of N.BspQI in DNA labelling and production of single-strand DNA. Protein Expr Purif. 2010 Feb; 69(2): 226-34. doi: 10.1016/j.pep.2009.09.003; WO 2023069948; and WO 1995009915 A1).

Although various methods for preparing single-stranded DNA already exist in the field, they suffer from drawbacks such as high cost, long time consumption, or relatively high residual dsDNA contamination. Therefore, there remains a need in the field for a simple and easy-to-operate method to prepare high-purity single-stranded DNA.

### SUMMARY

A first aspect of the present invention provides a method for preparing a circular single-stranded DNA, comprising the following steps:
1) providing a template DNA, wherein the template DNA is a circular double-stranded DNA comprising a target strand and a complementary strand;
2) adding gRNA and a Cas nicking enzyme to the template DNA, wherein the gRNA is complementary to a partial region of the complementary strand, and the Cas nicking enzyme forms, under the guidance of the gRNA, one or more nicks on the complementary strand; and
3) digesting the nicked complementary strand with an exonuclease to obtain a digestion product comprising the target strand in the form of the circular single-stranded DNA.

In one embodiment, the gRNA is sgRNA. In another specific embodiment, the Cas nicking enzyme is a Cas9 nicking enzyme, wherein for example, the Cas9 nicking enzyme is one or more selected from the group of: D10A, H840A, N863A, and N854A.

In one embodiment, the exonuclease is one or more selected from the group of: exonuclease T7, exonuclease ExoIII, and Lambda Exo.

In another embodiment, the template DNA is a plasmid. In more specific embodiments, the plasmid comprises an origin of replication and a gene of interest, and does not comprise a selection marker gene. The origin of replication is, for example, selected from a pUC origin of replication, a pMB1 origin of replication and derivatives thereof, a ColEl origin of replication, and an R6Kγ origin of replication.

In one embodiment, the one or more nicks are greater than or equal to two nicks.

In another embodiment, a ratio of the sequence length of the template DNA to the number of nicks is less than 10 kb per nick, preferably less than 5 kb per nicking site, further preferably less than 3 kb per nicking site, further more preferably less than 2 kb per nicking site, and most preferably less than 1 kb per nicking site.

In one embodiment, a molar ratio of the Cas nicking enzyme, the gRNA, and the template DNA is 3 : 3 : 1-12 : 12 : 1, preferably 4 : 4 : 1-11 : 11 : 1, and most preferably 5 : 5 : 1-10 : 10 : 1.

In one embodiment, the method further comprises the following step after step 3): purifying the digestion product to obtain a high-purity circular single-stranded DNA.

A second aspect of the present invention provides a method for preparing a linear single-stranded DNA, comprising the following step: cleaving the circular single-stranded (css) DNA obtained by the method according to the first aspect of the present invention to obtain a linear single-stranded (lss) DNA.

A third aspect of the present invention provides a kit for preparing a circular or linear single-stranded DNA, comprising a Cas nicking enzyme and an exonuclease.

In one embodiment, the Cas nicking enzyme is a Cas9 nicking enzyme, wherein for example, the Cas9 nicking enzyme is one or more selected from the group of: D10A, H840A, N863A, and N854A.

In one embodiment, the exonuclease is one or more selected from the group of: exonuclease T7, exonuclease ExoIII, and Lambda Exo.

In another embodiment, the kit further comprises a positive control template DNA and a positive gRNA. In yet another embodiment, the kit further comprises a template DNA and gRNA. In another embodiment, the kit further comprises a buffer solution. In another embodiment, the kit further comprises a reagent for purifying a circular or linear single-stranded DNA.

A fourth aspect of the present invention provides a circular or linear single-stranded DNA, comprising an origin of replication and a gene of interest, and not comprising a selection marker gene. In one embodiment, the origin of replication is selected from a pUC origin of replication, a pMB1 origin of replication and derivatives thereof, a ColE 1 origin of replication, and an R6Kγ origin of replication.

A fifth aspect of the present invention provides a composition comprising the circular or linear single-stranded DNA according to the fourth aspect of the present invention. In one embodiment, the composition is a pharmaceutical composition.

A sixth aspect of the present invention provides a digestion product comprising a target strand in the form of a circular single-stranded DNA, which is obtained by the method according to the first aspect of the present invention.

A seventh aspect of the present invention provides use of the circular or linear single-stranded DNA according to the fourth aspect of the present invention, or the composition according to the fifth aspect of the present invention, or the digestion product according to the sixth aspect of the present invention in gene therapy, gene recombination, DNA library construction, DNA origami, or DNA storage elements.

### Beneficial Technical Effects

In DNA sequences, 5'-NGG-3' or other PAM sequences are ubiquitous. Multiple gRNA sequences can be designed to nick double-stranded DNA according to experimental needs, which solves the problem of limited cleavage sites when a traditional nickase is used. In addition, multiple gRNAs are used in one enzyme digestion system for multi-point nicking, which not only enhances nicking efficiency but also significantly improves the digestion efficiency of the subsequent exonuclease. Therefore, the preparation of circular single-stranded or linear single-stranded DNA by using the method of the present invention is simple and easy to operate, highly efficient, and capable of yielding a high-purity single-stranded DNA.

### BRIEF DESCRIPTION OF THE DRAWINGS

FIG. 1: Closed circular single-stranded DNA (cssDNA) template plasmids (FIG. 1a: Nt.BspQI nicking site; and FIG. 1b: sgRNA nicking site)
FIG. 2: Digestion products of Nt.BspQI nickase
FIG. 3: Digestion products of Cas9_Nickase nickase
FIG. 4: Results of digestion of Nt.BspQI-nicked plasmids with exonuclease ExoIII
FIG. 5: Results of digestion of Cas9_Nickase-nicked plasmids with exonuclease ExoIII
FIG. 6: Results of template plasmid nicking
FIG. 7: Results of ExoIII digestion for 2 h
FIG. 8: Results of ExoIII digestion for 7 h in the Nt.BspQI group
FIG. 9: Purity comparison results of recovered closed circular single-stranded DNA (cssDNA) product
FIG. 10: Preparation efficiency of closed circular single-stranded DNA (cssDNA) with different numbers of sgRNAs

### DETAILED DESCRIPTION

Unless otherwise stated, all technical and scientific terms used herein have the meanings commonly understood by those of ordinary skill in the art. For the purpose of facilitating the understanding of the technical solutions provided herein, brief descriptions of some technical terms are provided below.

### Technical term

### ssDNA

### Single-stranded DNA (ssDNA)

### lssDNA

### Linear single-stranded DNA (lssDNA)

### cssDNA

### Closed circular single-stranded DNA (cssDNA)

In the present application, "template DNA" refers to a circular double-stranded DNA that serves as the starting material for preparing a single-stranded DNA. Similarly, "template plasmid" refers to a plasmid that serves as the starting material for preparing a single-stranded DNA. The template DNA or template plasmid is circular and double-stranded and comprises a target strand and a complementary strand.

"Circular", for example, "circular double-stranded DNA" or "circular single-stranded DNA", means that the DNA is covalently closed.

In the template DNA, the "target strand" and "complementary strand" refer to the DNA strand intended to be retained (i.e., used as the product) and the DNA strand to be nicked, respectively.

In the context of the present application, the terms "nicking enzyme" and "nicking endonuclease" (also referred to as NEase or nickase) are used interchangeably. A nicking enzyme recognizes a specific site in double-stranded DNA. However, unlike nucleases, the nicking enzyme only cleaves one strand of the DNA duplex at a position relative to the recognition site (doi: 10.3390/biom11101420). Taking the Cas9 protein as an example, Cas9 nuclease cleaves both strands of the DNA duplex, whereas Cas9 nicking enzyme is a mutant of the Cas9 nuclease (in which one or more of the two or more catalytic domains are inactivated via mutation, for example, by introducing an H840A mutation into the HNH domain or a D10A mutation into the Ruv C domain). This mutant retains the ability to bind to DNA based on gRNA specificity but can only cleave (or "nick") one strand of the DNA duplex, thereby generating a single-strand nick (DOI: 10.1101/gr.162339.113). For descriptions of various Cas nicking enzymes, see also WO 2023060256 A1.

The inventors of the present invention have found through research that the current technique of using a nickase such as Nb.BtsI to nick the non-target strand of a double-stranded plasmid, followed by digesting the nicked DNA strand with an exonuclease to ultimately obtain the target cssDNA is theoretically a simple and convenient method. However, this method has significant drawbacks in actual operation. For example, this method has very high requirements for cleavage sites. To only nick and digest the non-target strand of dsDNA in subsequent steps, it is necessary for the non-target strand to contain at least one target cleavage site (a condition that cannot be easily met by all dsDNA fragments). Moreover, all these target cleavage sites must be located on the non-target strand and completely absent from the target strand. Due to the above drawbacks, this method often requires modifying the dsDNA sequence to remove excess cleavage sites and retain only the cleavage site at the target position, which prolongs the production cycle. Due to the low nicking efficiency and digestion efficiency, a high level of residual dsDNA contamination is present in the final product, which impairs downstream experiments.

To overcome the above drawbacks and other deficiencies in the production of single-stranded RNA, a Cas nicking enzyme system (i.e., a Cas nicking enzyme and associated gRNA thereof) is used in the present invention to nick one strand of a circular double-stranded DNA template, generating one or more nicks in the strand. A Cas nicking enzyme is a mutant form of a Cas nuclease and can only cleave one strand of double-stranded DNA. Taking Cas9_D10A Cas nicking enzyme as an example, Cas9_D10A Nickase is a mutant form of the Cas9 nuclease, in which one active domain of the Cas9 nuclease is inactivated. Therefore, it can only rely on guide RNA to recognize the PAM site and produce single-stranded cleavage on the single-stranded DNA complementary to the guide RNA. The PAM site is a short sequence (5'-NGG-3') located at the 3' end of the target DNA. The guide RNA recognizes and binds to the complementary strand of the sequence, after which Cas9_D10A Nickase cleaves the recognized target DNA. In DNA sequences, PAM sites (e.g., 5'-NGG-3') are ubiquitous. Therefore, according to needs, a sequence of 20 nt or other appropriate lengths can be selected as the gRNA sequence upstream of multiple sites where the 3' end contains a PAM sequence (e.g., the GG sequence), which solves the problem of limited cleavage sites when a traditional nickase is used. In addition, multiple sgRNAs are used in one enzyme digestion system for multi-point nicking, which not only enhances nicking efficiency but also significantly improves the digestion efficiency of the subsequent exonuclease.

The present invention provides a method for preparing a circular single-stranded DNA, comprising the following steps:
1) providing (including but not limited to providing, obtaining, or generating) a template DNA, wherein the template DNA is a circular double-stranded DNA comprising a target strand and a complementary strand;
2) adding gRNA and a Cas nicking enzyme to the template DNA, wherein the gRNA is complementary to a partial region of the complementary strand, and the Cas nicking enzyme forms, under the guidance of the gRNA, one or more nicks on the complementary strand; and
3) digesting the nicked complementary strand with an exonuclease to obtain a digestion product comprising the target strand in the form of the circular single-stranded DNA.

It can be understood by those skilled in the art to which the present invention pertains that the gRNA used in the present invention may be sgRNA (single guide RNA), i.e., a guide RNA in a single-molecule form, or a guide RNA in a dual-molecule form. When gRNA is referred to in the present application, it shall be understood that sgRNA is also explicitly referred to, provided that the context permits. In one preferred embodiment, the gRNA is sgRNA. Methods for designing gRNAs, including sgRNAs, based on template sequences, such as preferred lengths and sequences thereof, are known in the art.

In the methods of the present invention, one or more gRNAs, preferably a plurality of gRNAs are preferably designed for the template DNA sequence, such that these gRNAs can find multiple nicking sites on the template DNA and generate multiple nicks. In preferred embodiments, a plurality of gRNAs, for example, 2-10 gRNAs or 2-5 gRNAs, for example 2, 3, 4, 5, 6, 7, 8, 9 or 10 gRNAs, are added to the template DNA in step 2). The number of Cas nicking enzymes may also be one or more, for example, 1, 2, 3, or 4.

The Cas nicking enzyme used in the present invention may be any Cas protein with nicking enzyme function, which can be guided by gRNA to a specific target DNA sequence (i.e., a target DNA sequence complementary to the gRNA) and can perform nicking enzyme function to cleave, and only cleave, one strand of double-stranded DNA. In preferred embodiments, the Cas nicking enzyme is a Cas9 nicking enzyme. The Cas9 nicking enzyme used in the present invention may be any Cas9 protein with nicking enzyme function, for example, including but not limited to those with mutations at one or more of the following amino acid sites: D10, G12, G17, E762, H840, N854, N863, H982, H983, A984, D986, and A987, for example, including but not limited to the following Cas9 nicking enzymes: D10A, H840A, N863A, and N854A. Various nicking enzymes can be used alone or in combination.

Non-limiting examples of Cas proteins include: Cas1, Cas1B, Cas2, Cas3, Cas4, Cas5, Cas6, Cas7, Cas8, Cas9 (also referred to as Csn1 and Csx12), Cas10, Cas12, Cas13, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or functional variants (e.g., codon-optimized) or modified forms thereof. In some embodiments, the Cas9 may be Cas9 derived from *Streptococcus pyogenes* or *Streptococcus pneumoniae.*

In some embodiments, the Cas nicking enzyme is a Cas12 nicking enzyme (Cas12a nicking enzyme) or a Cas13 nicking enzyme.

Any suitable exonuclease can be used in the present invention to enzymatically digest the nicked DNA strand, for example, including but not limited to the following exonucleases: exonuclease T7, exonuclease ExoIII, and Lambda Exo. Due to the properties of exonucleases, which act only or primarily on DNA with free 5' or 3' ends, and do not or rarely act on DNA without free 5' or 3' ends (e.g., covalently closed circular DNA), the exonucleases can digest and degrade the nicked DNA strand (which has nicks) in the template DNA duplex, without affecting, or significantly affecting, the other non-nicked, covalently closed circular DNA strand.

In one preferred embodiment, the template DNA is a plasmid, i.e., double-stranded plasmid DNA. The plasmid may be a conventional plasmid, comprising, for example, a backbone sequence, a promoter, an origin of replication, a selection marker gene (such as an antibiotic resistance gene or other selection marker genes), a multiple cloning site, a gene of interest sequence, etc.

In one preferred embodiment, the template plasmid comprises an origin of replication and a gene of interest, and does not comprise a selection marker gene. The origin of replication is, for example, selected from a pUC origin of replication, a pMB1 origin of replication and derivatives thereof, a ColEl origin of replication, and an R6Kγ origin of replication. In another preferred embodiment, the plasmid comprises only an origin of replication and a gene of interest, and does not comprise a selection marker gene; that is, the plasmid consists of an origin of replication and a gene of interest, and does not comprise a selection marker gene. In another preferred embodiment, the plasmid substantially consists of an origin of replication and a gene of interest, and does not comprise a selection marker gene. The preparation method for the plasmid can be referred to in PCT/CN2021/133141, which is incorporated herein by reference in its entirety.

In one embodiment, the one or more nicks are greater than or equal to two nicks, for example, 2-10 nicks or 2-5 nicks, for example, 2, 3, 4, 5, 6, 7, 8, 9, or 10 nicks. In another embodiment, a ratio of the sequence length of the template DNA to the number of nicks is less than 10 kb per nick, preferably less than 5 kb per nick, further preferably less than 3 kb per nick, further more preferably less than 2 kb per nick, and most preferably less than 1 kb per nick.

In one embodiment, a molar ratio of the Cas nicking enzyme (e.g., Cas9 nicking enzyme), the gRNA, and the template DNA is 3 : 3 : 1-12 : 12 : 1, preferably 4 : 4 : 1-11 : 11 : 1, and most preferably 5 : 5 : 1-10 : 10 : 1.

In one preferred embodiment, the method further comprises the following step after step 3): purifying the digestion product to obtain a high-purity circular single-stranded DNA. High purity refers to the absence of impurities, or the substantial absence of impurities, or the absence of a significant amount of impurities. For example, based on mass, at least 70%, 80%, 85%, 90%, 95%, 96%, 97%, 98%, 99%, or 100% of the total DNA in the final product obtained after purification is circular single-stranded DNA. After digesting the nicked complementary strand with exonuclease, suitable purification methods may be employed to remove impurities other than circular single-stranded DNA from the digestion product. The purification methods include, but are not limited to, alcohol precipitation, column recovery, magnetic bead recovery, phenol/chloroform/isoamyl alcohol recovery, etc.

The present invention further provides a digestion product comprising a target strand in the form of a circular single-stranded DNA, which is obtained by the above method of the present invention. The digestion product comprises a circular single-stranded DNA, a product of the nicked DNA strand after exonuclease digestion, gRNA, and a Cas nicking enzyme.

The present invention further provides a method for preparing a linear single-stranded DNA, comprising the following step: cleaving the circular single-stranded (css) DNA obtained by the method of the present invention to obtain a linear single-stranded (lss) DNA. Any method known in the art for cleaving a circular single-stranded DNA to obtain a linear single-stranded DNA can be used in this step. In preferred embodiments, cleavage is performed at a selected site, for example, using a Cas9 system capable of cleaving single-stranded DNA for site-specific cleavage.

The present invention further provides a kit for preparing a circular or linear single-stranded DNA, the kit comprises a Cas nicking enzyme and an exonuclease. In one embodiment, the kit further comprises a positive control template DNA and a positive gRNA, which are used to confirm that components comprised in the kit can produce a circular or linear single-stranded DNA in a predetermined manner (for example, in the manner described in the instructions accompanying the kit). In yet another embodiment, the kit further comprises a template DNA and gRNA. In another embodiment, the kit further comprises a buffer solution to facilitate the nicking reaction and/or the exonuclease digestion reaction. In another embodiment, the kit further comprises a reagent for purifying a circular or linear single-stranded DNA.

The present invention further provides a circular or linear single-stranded DNA, comprising an origin of replication and a gene of interest, and not comprising a selection marker gene. In one embodiment, the origin of replication is selected from a pUC origin of replication, a pMB1 origin of replication and derivatives thereof, a ColE1 origin of replication, and an R6Kγ origin of replication. In another preferred embodiment, the plasmid comprises only an origin of replication and a gene of interest, and does not comprise a selection marker gene; that is, the plasmid consists of an origin of replication and a gene of interest, and does not comprise a selection marker gene. In another preferred embodiment, the plasmid substantially consists of an origin of replication and a gene of interest, and does not comprise a selection marker gene. The preparation method for the plasmid can be referred to in PCT/CN2021/133141, which is incorporated herein by reference in its entirety.

The present invention further provides a composition comprising the circular or linear single-stranded DNA described above. In one embodiment, the composition is a pharmaceutical composition, which further comprises a pharmaceutically conventional excipient, including but not limited to a filler, a disintegrant, a lubricant, a flavouring agent, a colorant, etc.

The circular or linear single-stranded DNA, the digestion product comprising the circular single-stranded DNA, and the composition comprising the circular or linear single-stranded DNA according to the present invention can be suitably applied in gene therapy, gene recombination (e.g., gene knock-in), construction of DNA libraries (e.g., DNA screening libraries), DNA origami, or DNA storage elements.

All references cited in the present application are incorporated herein by reference as if each individual reference were specifically and individually indicated to be incorporated by reference herein.

The technical solutions of the present invention are further described in detail below through examples and in conjunction with the accompanying drawings. Unless otherwise stated, the methods and materials of the examples described below are all conventional products commercially available. It will be understood by those skilled in the art that the methods and materials described below are merely exemplary, and should not be construed as limiting the scope of the present invention in any way. The scope of the present invention is limited solely by the claims.

### General Experimental Method

(1) A template plasmid for the preparation of cssDNA is constructed, and specific sgRNAs are designed and synthesized based on the full-length sequence of the plasmid. The sgRNAs guide a Cas9_Nickase nickase to perform site-specific nicking at multiple sites on the template plasmid. As a control, the plasmid also carries 3 tandem Nt.BspQI cleavage sites located on the same DNA strand, which are used to enhance the nicking efficiency of the nickase Nt.BspQI.
(2) In the experimental group, the template plasmid is digested with Cas9_Nickase to obtain an open circular DNA with multiple nicks on one strand. In the control group, the template plasmid is digested with Nt.BspQI to obtain an open circular DNA with a single nick on one strand. The open circular DNA derived from Cas9_Nickase and the open circular DNA derived from Nt.BspQI are each digested with the exonuclease ExoIII to obtain the target circular single-stranded DNA, and purity comparison is performed between the two products.

### Examples

### Example 1: Construction of cssDNA template plasmid and design of sgRNA

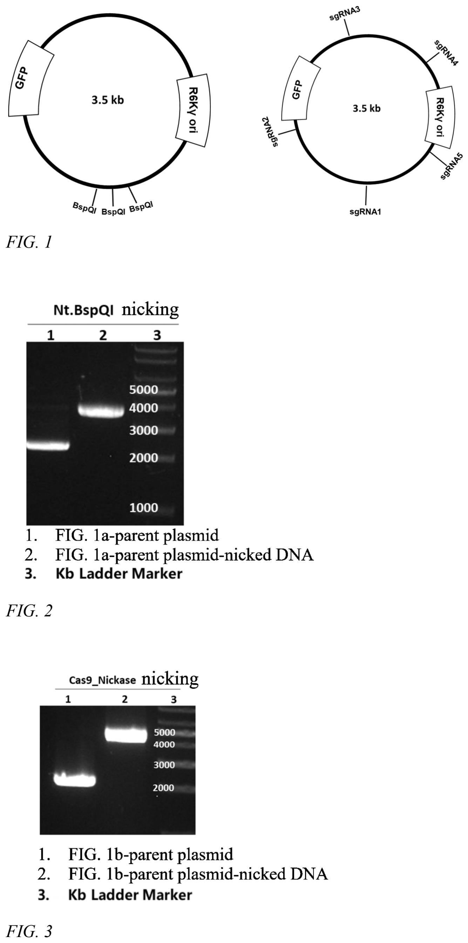

A template plasmid pMF5-GFP-BspQI (FIG. 1b) for the preparation of cssDNA was constructed, with a full length of 3553 bp. The plasmid was synthesized by the Gene Synthesis Department of GenScript Biotech Corp., Nanjing. The sequence is shown in the sequence listing. The full-length sequence of the pMF5-GFP-BspQI plasmid was imported into an sgRNA design website (http://crispor.tefor.net/), and multiple sgRNA sequences were selected based on the desired target sites and the full-length sequence of the plasmid. A total of 5 sgRNAs were selected in this example, each spaced approximately 700 bp apart. The sequences are shown in the sequence listing. These sgRNAs were synthesized by the Nucleic Acid Chemistry Department of GenScript Biotech Co., Ltd., Nanjing.

Meanwhile, as the template plasmid for the control group *Nt.BspQI* (FIG. 1a), the sense strand of pMF5-GFP-BspQI carries three closely spaced *Nt.BspQI* cleavage sites, which are used to improve the nicking efficiency of the *Nt.BspQI* nickase in the control group.

### Example 2: Preparation of cssDNA using nickase Nt.BspQI/Cas9_ Nickase

### 1. Preparation of nicked plasmids

### 1.1 Control group: A nicked plasmid was prepared using nickase Nt.BspQI.

In the control group, the traditional nickase *Nt.BspQI* was selected for the preparation of a nicked plasmid. The pMF5-GFP-BspQI plasmid was treated with *Nt.BspQI* to obtain an open circular DNA with a single nick on one strand. The enzyme digestion system is as shown in Table 1*. Nt. BspQI* was purchased from New England Biolabs, Cat. No.: R0644S.

**Table 1 Nt.BspQI enzyme digestion system**

| System | Volume |
|---|---|
| Nt.BspQI | 50 µl |
| 10×NEBuffer r3.1 | 500 µl |
| pMF5-GFP-BspQI plasmid template (1000 ng/µl) | 500 µl |
| H₂O | 3950 µl |

The enzyme digestion reaction was performed at 500 rpm for 2.5 h at 50°C. 1 µl of the sample was taken for gel electrophoresis verification. The enzyme digestion result is as shown in FIG. 2, confirming that the enzyme digestion reaction was complete. Heat inactivation was performed at 80°C for 20 min. 20 µl of the inactivated sample was stored in a 4°C refrigerator, which was used as a control for subsequent gel electrophoresis.

Recovery of enzyme-digested samples using phenol/chloroform/isoamyl alcohol:
1) An equal volume of phenol/chloroform/isoamyl alcohol (25 : 24 : 1) was added, vortexed for 10 s, and then centrifuged at 5000 g for 5 min.
2) The upper aqueous phase was transferred to a new tube, and an equal volume of chloroform/isoamyl alcohol (24 : 1) was added. The mixture was vortexed for 10 s and centrifuged at 5000 g for 5 min.
3) Step 2 was repeated.
4) The upper aqueous phase was transferred to a new tube. 1/10 volume of 3 M sodium acetate and 2.5 volumes of ice-cold ethanol were added, mixed well, and incubated at -20°C for 1 h.
5) Centrifugation was performed at 10,000 rpm for 10 min.
6) The supernatant was discarded, and the precipitate was gently washed with 2000 µL of 75% ice-cold ethanol, followed by evaporation of residual ethanol.
7) An appropriate amount of DNase/RNase-free H₂O was added to dissolve the plasmid.

### 1.2. Experiment group: A nicked plasmid was prepared using nickase Cas9_Nickase.

To compare the nicking efficiency with that of the traditional nickase, the pMF5-GFP-BspQI plasmid in the experimental group was treated with Cas9_Nickase to obtain an open circular DNA with 5 nicks on one strand. The enzyme digestion system is as shown in Table 2. Engenpy Cas9_Nickase was purchased from New England Biolabs, Cat. No.: M0650S.

**Table 2 Cas9_Nickase enzyme digestion system**

| System | Volume |
|---|---|
| 1 µM Cas9_Nickase | 2.28 ml |
| 10×NEBuffer r3.1 | 3.6 ml |
| 10 pM sgRNA | 0.228 ml*5 |
| H₂O | 28.48 ml |
| Reaction volume | 35.5 ml |
| Reaction condition | 25°C, 10 min |
| pMF5-GFP-BspQI plasmid template (1000 ng/µl) | 0.5 ml |
| Reaction condition | 37°C, 2.5 h |

5 µl of the sample was taken, and 1 µl of proteinase K was added. The mixture was left standing at room temperature for 15 min, followed by gel electrophoresis verification. The enzyme digestion result is as shown in FIG. 3, confirming that the enzyme digestion reaction was complete. 100 µl of proteinase K was added to the total reaction. The mixture was left standing at room temperature for 15 min. Heat inactivation was performed at 95°C for 10 min. 20 µl of the inactivated sample was stored in a 4°C refrigerator, which was used as a control for subsequent gel electrophoresis.

The step of recovering the enzyme-digested samples using phenol/chloroform/isoamyl alcohol was the same as that described in 1.1.

### 2. Preparation of cssDNA

The nicked plasmids prepared in step 1.1 and step 1.2 were digested with exonuclease *ExoIII,* and purity comparison was performed between the products obtained after *ExoIII* digestion of the two nicked plasmids from different sources. *ExoIII* was purchased from New England Biolabs, Cat. No.: M0206L.

### 2.1 Digestion of nicked plasmid derived from Nt.BspQI with exonuclease ExoIII

The nicked plasmid prepared in step 1.1 was digested with exonuclease *ExoIII.* The digestion system is as shown in Table 3.

**Table 3 Digestion of nicked plasmid system derived from Nt.BspQI with ExoIII**

| System | Volume |
|---|---|
| ExoIII | 3 µl |
| 10×NEBuffer 1 | 30 µl |
| *Nt.BspQI* nicked plasmid template (1550 ng/µl) | 19 µl |
| H₂O | 248 µl |

At 0.25 h, 0.5 h, 0.75 h, 1 h, 1.5 h, 2 h, 3 h, 4 h, 5 h, 7 h, and 24 h, 20 µl of samples were separately taken, and 11 mM EDTA was added to each sample. The samples were heat-inactivated at 70°C for 20 min, and temporarily stored in a 4°C refrigerator for gel electrophoresis verification after the digestion period was completed.

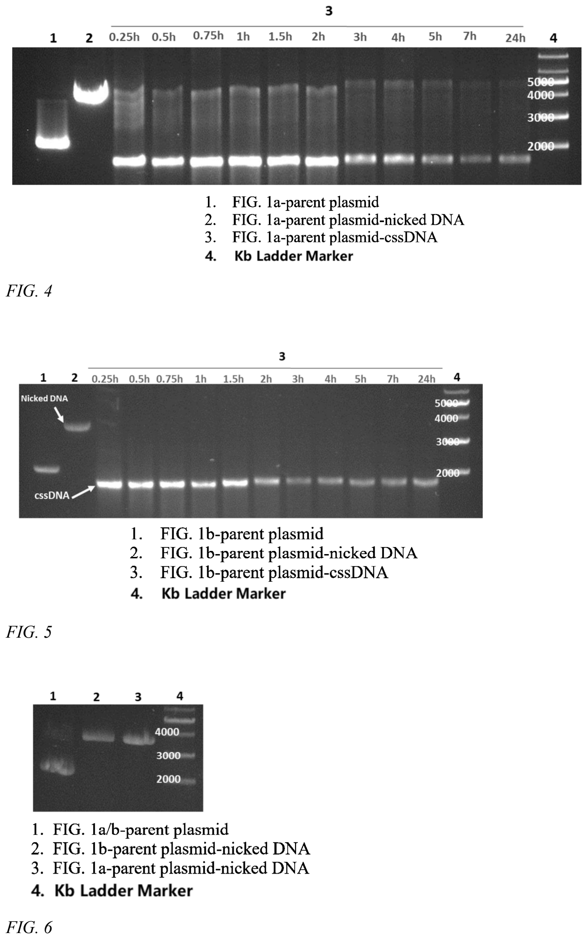

The digestion results are as shown in FIG. 4. Significant residual nicked plasmid was still observed after 5 h of digestion; even after 24 h of digestion, the nicked plasmids remained incompletely digested. Furthermore, the content of cssDNA gradually decreased as the digestion time prolonged. The gel electrophoresis results were analysed using a Tanon GIS series digital gel image processing system. The specific ratios of cssDNA and residual nicked plasmid are as shown in Table 4.

**Table 4 Ratios of cssDNA and residual nicked plasmid in Nt.BspQI-nicked ExoIII digestion product**

| Digestion time | 0.25 h | 0.5 h | 0.75 h | 1 h | 1.5 h | 2 h | 3 h | 4 h | 5 h | 7 h | 24 h |
|---|---|---|---|---|---|---|---|---|---|---|---|
| Ratio of cssDNA (%) | 50.48 | 81.28 | 62.17 | 76.99 | 76.76 | 78.62 | 74.97 | 73.6 | 69.65 | 69.14 | 74.45 |
| Ratio of residual nicked plasmid (%) | 49.52 | 18.72 | 37.83 | 23.01 | 23.24 | 21.38 | 25.03 | 26.4 | 30.35 | 30.86 | 25.55 |

### 2.2 Digestion of nicked plasmid derived from Cas9_Nickase with exonuclease ExoIII

The nicked plasmid prepared in step 1.2 was digested with exonuclease *ExoIII.* The digestion system is as shown in Table 5.

**Table 5 Digestion of nicked plasmid system derived from Cas9_Nickase with ExoIII**

| System | Volume |
|---|---|
| ExoIII | 3 µl |
| 10×NEBuffer 1 | 30 µl |
| Cas9_Nickase nicked plasmid template (2921.6 ng/µl) | 10 µl |
| H₂O | 257 µl |

At 0.25 h, 0.5 h, 0.75 h, 1 h, 1.5 h, 2 h, 3 h, 4 h, 5 h, 7 h, and 24 h, 20 µl of samples were separately taken, and 11 mM EDTA was added to each sample. The samples were heat-inactivated at 70°C for 20 min, and temporarily stored in a 4°C refrigerator for subsequent gel electrophoresis verification.

The digestion results are shown in FIG. 5. A faint amount of residual nicked plasmid was observed at 0.25 h of digestion. At 0.5 h of digestion, the nicked plasmid was completely digested, and no residual nicked plasmid was detected. After 24 h of digestion, the content of cssDNA slightly decreased as the digestion time prolonged, but the band remained stable. The gel electrophoresis results were analysed using a Tanon GIS series digital gel image processing system. The specific ratios of cssDNA and residual nicked plasmid are as shown in Table 6.

**Table 6 Ratios of cssDNA and residual nicked plasmid in Cas9_Nickase-nicked ExoIII digestion product**

| Digestion time | 0.25 h | 0.5 h | 0.75 h | 1 h | 1.5 h | 2 h | 3 h | 4 h | 5 h | 7 h | 24 h |
|---|---|---|---|---|---|---|---|---|---|---|---|
| Ratio of cssDNA (%) | 100 | 100 | 100 | 100 | 100 | 100 | 100 | 100 | 100 | 100 | 100 |
| Ratio of residual nicked plasmid (%) | 0 | 0 | 0 | 0 | 0 | 0 | 0 | 0 | 0 | 0 | 0 |

In comparison between the traditional nickase *Nt.BspQI* and the Cas9_Nickase of the present invention, selecting the sgRNA-dependent nickase Cas9_Nickase to perform multi-point nicking on the template plasmid, followed by digesting the nicked plasmid with exonuclease *ExoIII,* enables the production of higher-purity cssDNA in a shorter time. Meanwhile, as the digestion time of exonuclease is greatly shortened, the degradation of the product cssDNA by exonuclease is also slight, which effectively improves the preparation efficiency of cssDNA.

### Example 3: Comparison of recovery rates of cssDNAs prepared in the Nt.BspQI group/Cas9_Nickase group

In this example, the recovery rates of cssDNAs prepared in the Nt.BspQI group/Cas9_Nickase group were compared, with the product purity used as the recovery standard.

### 3.1 Preparation of nicked plasmids

Nicked plasmids were prepared using the nickases *Nt.BspQI* and Cas9_Nickase, respectively. The initial input amount of the template plasmid was 300 µg for both groups.

**Table 7 Nt.BspQI enzyme digestion system**

| System | Volume |
|---|---|
| Nt.BspQI | 30 µl |
| 10×NEBuffer r3.1 | 300 µl |
| pMF5-GFP-BspQI plasmid template (1000 ng/µl) | 300 µl |
| H₂O | 2370 µl |

The enzyme digestion reaction was performed at 500 rpm for 3 h at 50°C. 1 µl of the sample was taken for gel electrophoresis verification. The enzyme digestion result is as shown in Lane 2 of FIG. 6, confirming that the enzyme digestion reaction was complete. Heat inactivation was performed at 80°C for 20 min. 20 µl of the inactivated sample was stored in a 4°C refrigerator, which was used as a control for subsequent gel electrophoresis.

**Table 8 Cas9_Nickase enzyme digestion system**

| System | Volume |
|---|---|
| 20 µl Cas9_Nickase | 0.0683 ml |
| 10×NEBuffer r3.1 | 2 ml |
| 10 pM sgRNA | 0.1366 ml*5 |
| H₂O | 16.9487 ml |
| Reaction volume | 19.7 ml |
| Reaction condition | 25°C, 10 min |
| pMF5-GFP-BspQI plasmid template (1000 ng/µl) | 0.3 ml |
| Reaction condition | 37°C, 3 h, 500 rpm |

3 µl of the sample was taken, and 1 µl of proteinase K was added. The mixture was left standing at room temperature for 15 min, followed by gel electrophoresis verification. The enzyme digestion result is as shown in Lane 3 of FIG. 6, confirming that the enzyme digestion reaction was complete. 100 µl of proteinase K was added to the total reaction. The mixture was left standing at room temperature for 15 min. Heat inactivation was performed at 95°C for 10 min. 20 µl of the inactivated sample was stored in a 4°C refrigerator, which was used as a control for subsequent gel electrophoresis.

### 3.2 Recovery of nicked plasmids

Recovery of enzyme-digested samples using phenol/chloroform/isoamyl alcohol:
1) An equal volume of phenol/chloroform/isoamyl alcohol (25 : 24 : 1) was added, vortexed for 10 s, and then centrifuged at 5000 g for 5 min.
2) The upper aqueous phase was transferred to a new tube, and an equal volume of chloroform/isoamyl alcohol (24 : 1) was added. The mixture was vortexed for 10 s and centrifuged at 5000 g for 5 min.
3) Step 2 was repeated.
4) The upper aqueous phase was transferred to a new tube. 1/10 volume of 3 M sodium acetate and 2.5 volumes of ice-cold ethanol were added, mixed well, and incubated at -20°C for 1 h.
5) Centrifugation was performed at 10,000 rpm for 10 min.
6) The supernatant was discarded, and the precipitate was gently washed with 2000 µL of 75% ice-cold ethanol, followed by evaporation of residual ethanol.
7) For both groups, 200 µl of DNase/RNase-free H₂O was added to dissolve the products.
8) From each group, 1 µl of sample was taken, and the concentration was determined using a NanoDrop^{™} One Microvolume UV-Vis Spectrophotometer.

**Table 9 Recovery efficiency of nicked plasmids**

| **Group** | **Recovery concentration (ng/µl)** | **A260/280** | **A260/230** | **Total recovery amount (µg)** | **Recovery rate (%)** |
|---|---|---|---|---|---|
| **Nt.BspQI group** | 1186 | 1.87 | 2.38 | 237.2 | 79.1 |
| **Cas9_Nickase group** | 1423.4 | 1.91 | 2.36 | 284.68 | 94.8 |

### 3.3 Preparation of cssDNA

The recovered products from both groups in step 3.2 were separately digested with exonuclease *ExoIII,* and all the recovered products were added into the digestion system. The digestion system is as shown in Table 10. Finally, the recovery of cssDNA was performed with the product purity as the recovery standard.

**Table 10 ExoIII digestion system**

| System | Volume |
|---|---|
| ExoIII | 30 µl |
| 10×NEBuffer 1 | 300 µl |
| Recovered product from step 3.2 | 200 µl |
| H₂O | 2470 µl |

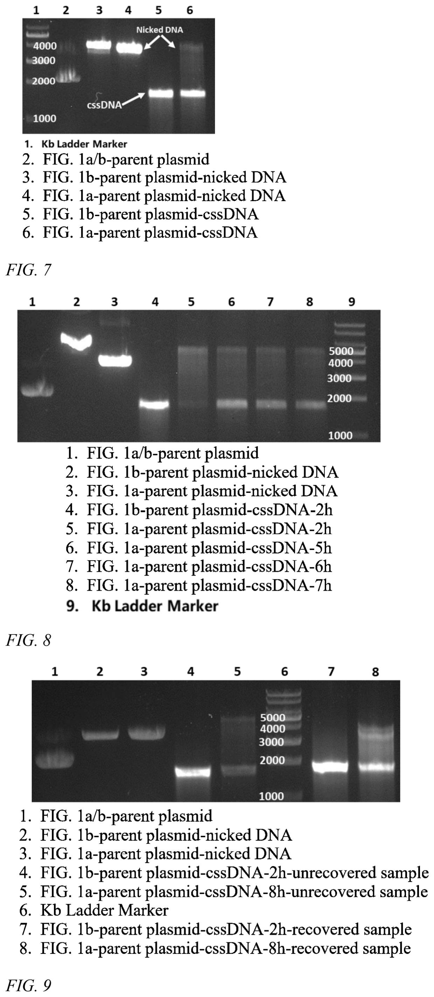

The digestion was performed at 37°C at 500 rpm. At 2 h of digestion, 1 µl of sample was taken from each group for agarose gel electrophoresis verification. The results are as shown in FIG. 7. In the Cas9_Nickase group, after 2 h of digestion, the nicked DNA band in Lane 3 was converted into a higher-purity cssDNA band in Lane 5. In contrast, in the Nt.BspQI group, after 2 h of digestion, the cssDNA in Lane 6 still exhibited significant contamination from nicked DNA bands. The products of the Cas9_Nickase group were stored temporarily in a 4°C refrigerator.

The Nt.BspQI group continued digestion. The samples were taken at 5 h, 6 h, and 7 h for gel electrophoresis to observe the digestion status. The results are as shown in FIG. 8. After 7 h of digestion, the residual nicked DNA was still not completely digested, while the content of the target product cssDNA decreased as the digestion time prolonged.

The Nt.BspQI group continued digestion until 8 h, and then the products were subjected to phenol/chloroform/isoamyl alcohol recovery together with the 2 h-digested products of the Cas9_Nickase group. After recovery, the recovery rates were compared, and agarose gel electrophoresis was performed again to verify the product purity. The recovery rates are shown in Table 11, and the electrophoresis results are shown in FIG. 9.

**Table 11 Comparison of cssDNA recovery efficiency**

| **Group** | **Recovery concentration (ng/µl)** | **A260/280** | **A260/230** | **Recovery volume (µl)** | **Total recovery amount (µg)** | **Total recovery rate (%)** |
|---|---|---|---|---|---|---|
| **Nt.BspQI group** | 96 | 1.77 | 2.14 | 50 | 4.8 | 1.6 |
| **Cas9_Nick ase group** | 1232 | 1.91 | 2.36 | 50 | 61.6 | 20.5 |

From the digestion results of the Nt.BspQI group, it can be seen that in addition to digesting and degrading nicked ssDNA, exonuclease ExoIII also exhibits a certain degradation effect on circular ssDNA. When Nt.BspQI is selected as the nickase to nick the template plasmid at a single site, the length of the ssDNA fragment to be digested is excessively long. As a result, the digestion efficiency of exonuclease ExoIII fails to reach the ideal state, leading to low product purity and significant contamination. Furthermore, attempting to improve product purity by prolonging the digestion time results in degradation of the target product cssDNA, ultimately reducing the final recovery efficiency.

Compared to the Nt.BspQI group, the Cas9_Nickase group significantly shortens the length of the fragment to be digested through multi-site nicking. This not only enhances the digestion efficiency of exonuclease ExoIII within a short period but also effectively reduces the digestion time while improving product purity. As a result, the recovery efficiency of the final product is much higher than that of the Nt.BspQI group.

### Example 4: Design of sgRNA in preparation of cssDNA using Cas9_Nickase

Different sgRNAs were designed using the plasmid sequence in FIG. 1b (right) as the template, with a total of 4 groups designed. The nicking sites generated by Cas9_Nickase-sgRNA and the lengths of spacer sequences between adjacent nicking sites in each group are as shown in Table 12. The preparation of nicked DNA and cssDNA was performed for each of the 4 groups, following the same procedure as in Example 2.

**Table 12 Number of nicking sites and length of each fragment**

| **Group** | **Number of sgRNAs** | **Number of nicks generated** | **Length of nicked strand** |
|---|---|---|---|
| **1** | 5 | 5 | 0.7 kb |
| **2** | 3 | 3 | 1.2 kb |
| **3** | 2 | 2 | 1.7 kb |
| **4** | 1 | 1 | 3.5 kb |

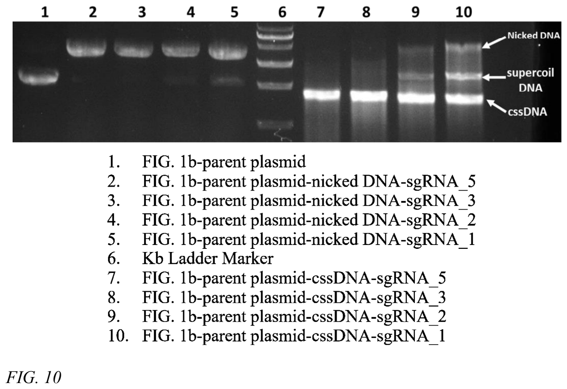

The preparation results are as shown in FIG. 10. Lanes 2-5 represented the preparation results of nicked DNA when the number of sgRNAs was 5, 3, 2, and 1, respectively; Lanes 7-10 represented the preparation results of cssDNA when the number of sgRNAs was 5, 3, 2, and 1, respectively.

As shown in the results of Lane 9 and Lane 10, when 2 or 1 sgRNA was selected for nicking followed by ExoIII digestion, 3 forms of DNA were observed in the products. From top to bottom, the bands include: Product 1: nicked DNA not completely digested by ExoIII; Product 2: residual parental plasmid DNA; and Product 3: the target product cssDNA, which accounts for the majority proportion. The proportions of Product 1 and Product 2 in the 1-sgRNA group (Lane 10) were significantly higher than those in the 2-sgRNA group (Lane 9). The results show that when designing sgRNAs based on the actual size of the template plasmid, both the number of sgRNAs and the length of nicked strands critically affect the final preparation efficiency and purity of cssDNA. Specifically, the number of sgRNAs should be no less than 2, and the length of nicked strands should be less than 1.7 kb.

The embodiments of the present invention are not limited to those described in the above examples. Without departing from the spirit and scope of the present invention, those of ordinary skill in the art can make various changes and improvements to the present invention in form and detail, and all such changes and improvements are deemed to fall within the protection scope of the present invention.

pMF5-GFP-BspQI (SEQ ID NO: 1):

TACCGttcgtataatgtatgctatacgaacggtaCTGCAGtgtcagccgttaagtgttcctgtgtcactcaaaatt
gctttgagaggctctaagggcttctcagtgcgttacatccctggcttgttgtccacaaccgttaaaccttaaaagctttaaa
agccttatatattcttttttttcttataaaacttaaaaccttagaggctatttaagttgctgatttatattaattttattgttcaaacat
gagagcttagtacgtgaaacatgagagcttagtacgttagccatgagagcttagtacgttagccatgagggtttagttcg
ttaaacatgagagcttagtacgttaaacatgagagcttagtacgtgaaacatgagagcttagtacgtactatcaacaggtt
gaactgctgatcttcagatcGGATCCGGTTTCCTTGAGTGGCAGGCCAGGCCTGGCC
GTGAACGTTCACTGAAATCATGGCCTCTTGGCCAAGATTGATAGCTTGT
GCCTGTCCCTGAGTCCCAGTCCATCACGAGCAGCTGGTTTCTAAGATGC
TATTTCCCGTATAAAGCATGAGACCGTGACTTGCCAGCCCCACAGAGCC
CCGCCCTTGTCCATCACTGGCATCTGGACTCCAGCCTGGGTTGGGGCAA
AGAGGGAAATGAGATCATGTCCTAACCCTGATCCTCTTGTCCCACAGAT
ATCCAGAACCCTGACCCTGCCGTGGCTACTAACTTCAGCCTGCTGAAGC
AGGCTGGAGACGTGGAGGAGAACCCTGGACCTATGGCTCTTCCGGTGA
CCGCTCTTCTTCTCCCGCTGGCTCTTCTGCTCCATGCTGCGCGACCGGAC
ATTCAAATGACCCAAACTACATCTTCTTTGTCTGCTTCCCTGGGTGATC
GGGTGACCATTTCATGTAGAGCCAGTCAGGATATAAGTAAATACCTGA
ATTGGTATCAACAGAAGCCAGATGGAACAGTAAAGTTGCTGATTTATC
ACACCTCACGATTGCACTCAGGTGTTCCATCCAGGTTTTCAGGAAGTGG
CTCTGGAACTGACTATTCTTTGACAATTAGTAACTTGGAACAAGAAGAT
ATTGCTACGTATTTCTGCCAACAGGGTAACACTCTTCCTTATACTTTTGG
CGGCGGCACTAAATTGGAGATTACAGGGGGCGGAGGCTCCGGTGGCGG
AGGATCTGGCGGGGGTGGGAGCGAAGTTAAGTTGCAGGAAAGTGGGC
CTGGGTTGGTTGCACCCAGCCAAAGCCTCTCAGTAACCTGCACGGTTTC
CGGAGTCAGTCTCCCAGACTACGGAGTATCATGGATCCGCCAGCCACC
ACGGAAGGGGTTGGAGTGGCTGGGAGTCATCTGGGGATCAGAAACGAC
GTATTACAATTCCGCCCTCAAAAGCCGGCTGACTATTATAAAAGACAA
CAGCAAGTCACAAGTGTTCCTGAAAATGAATAGTCTCCAGACGGATGA
CACGGCCATTTACTATTGCGCAAAGCACTATTACTACGGTGGGTCTTAT
GCAATGGACTACTGGGGTCAAGGGACCTCAGTCACAGTTTCCAGTACT
ACAACTCCAGCGCCGCGACCCCCGACGCCAGCACCAACGATAGCCTCT
CAGCCGCTTTCCTTGAGGCCGGAGGCTTGCAGGCCCGCGGCCGGGGGC
GCAGTTCATACCAGAGGGCTTGATTTTGCCTGTGACATCTACATATGGG
CACCGCTCGCAGGAACTTGTGGTGTATTGTTGCTTTCACTGGTGATAAC
CCTCTATTGCAAACGCGGACGGAAGAAGCTCCTCTATATTTTTAAGCAG

CCGTTTATGCGCCCGGTACAAACGACGCAGGAGGAGGATGGCTGTTCC
TGTAGATTTCCTGAAGAGGAAGAGGGTGGATGCGAGCTCAGAGTCAAA
TTCTCACGCTCTGCGGACGCTCCAGCCTACAAACAGGGTCAGAACCAG
TTGTACAACGAGCTTAATTTGGGACGCCGCGAAGAGTATGATGTCCTTG
ACAAACGCAGGGGCAGGGACCCGGAAATGGGCGGCAAGCCACGGCGC
AAGAACCCGCAGGAGGGTCTGTATAACGAGCTTCAGAAGGATAAGATG
GCCGAGGCCTACTCAGAAATAGGAATGAAGGGAGAGCGGCGGAGAGG
CAAGGGCCACGATGGTCTGTATCAGGGGCTTTCCACGGCAACCAAAGA
CACATATGACGCTCTCCACATGCAGGCCCTCCCCCCGCGGGAGGGCAG
GGGATCTCTCCTTACTTGTGGCGACGTGGAGGAGAACCCCGGCCCCAG
CAAGGGCGAGGAGCTGTTCACCGGGGTGGTGCCCATCCTGGTCGAGCT
GGACGGCGACGTAAACGGCCACAAGTTCAGCGTGCGCGGCGAGGGCG
AGGGCGATGCCACCAACGGCAAGCTGACCCTGAAGTTCATCTGCACCA
CCGGCAAGCTGCCCGTGCCCTGGCCCACCCTCGTGACCACCCTGACCTA
CGGCGTGCAGTGCTTCAGCCGCTACCCCGACCACATGAAGCGCCACGA
CTTCTTCAAGTCCGCCATGCCCGAAGGCTACGTCCAGGAGCGCACCATC
AGCTTCAAGGACGACGGCACCTACAAGACCCGCGCCGAGGTGAAGTTC
GAGGGCGACACCCTGGTGAACCGCATCGAGCTGAAGGGCATCGACTTC
AAGGAGGACGGCAACATCCTGGGGCACAAGCTGGAGTACAACTTCAAC
AGCCACAACGTCTATATCACCGCCGACAAGCAGAAGAACGGCATCAAG
GCCAACTTCAAGATCCGCCACAACGTGGAGGACGGCAGCGTGCAGCTC
GCCGACCACTACCAGCAGAACACCCCCATCGGCGACGGCCCCGTGCTG
CTGCCCGACAACCACTACCTGAGCACCCAGTCCGTGCTGAGCAAAGAC
CCCAACGAGAAGCGCGATCACATGGTCCTgCTGGAgTTCGTGACCGCCG
CCGGGATCACTtgaTGATCATAATCAAGCCATATCACATCTGTAGAGGTT
TACTTGCTTTAAAAAACCTCCACACCTCCCCCTGAACCTGAAACATAAA
ATGAATGCAATTGTTGTTGTTAACTTGTTTATTGCAGCTTATAATGGTTA
CAAATAAAGCAATAGCATCACAAATTTCACAAATAAAGCATTTTTTTCA
CTGCATTCTAGTTGTGGTTTGTCCAAACTCATCAATGTATCTTATCATGT
CTGGATCACCAGCTGAGAGACTCTAAATCCAGTGACAAGTCTGTCTGCC
TATTCACCGATTTTGATTCTCAAACAAATGTGTCACAAAGTAAGGATTC
TGATGTGTATATCACAGACAAAACTGTGCTAGACATGAGGTCTATGGA
CTTCAAGAGCAACAGTGCTGTGGCCTGGAGCAACAAATCTGACTTTGC
ATGTGCAAACGCCTTCAACAACAGCATTATTCCAGAAGACACCTTCTTC

CCCAGCCCAGGTAAGGGCAGCTTTGGTGCCTTCGCAGGCTGTTTCCTTG
CTTCAGGAATGGCCATCTAGAGTCGACGCATGC

## Claims

1. A method for preparing a circular single-stranded DNA, **characterized in that** the method comprises the following steps:
1) providing a template DNA, wherein the template DNA is a circular double-stranded DNA comprising a target strand and a complementary strand;
2) adding gRNA and a Cas nicking enzyme to the template DNA, wherein the gRNA is complementary to a partial region of the complementary strand, and the Cas nicking enzyme forms, under the guidance of the gRNA, one or more nicks on the complementary strand; and
3) digesting the nicked complementary strand with an exonuclease to obtain a digestion product comprising the target strand in the form of the circular single-stranded DNA.

2. The method according to claim 1, **characterized in that** the gRNA is sgRNA.

3. The method according to claim 1 or 2, **characterized in that** the Cas nicking enzyme is a Cas9 nicking enzyme.

4. The method according to claim 3, **characterized in that** the Cas9 nicking enzyme is one or more selected from the group of: D10A, H840A, N863A, and N854A.

5. The method according to any one of the foregoing claims, **characterized in that** the exonuclease is one or more selected from the group of: exonuclease T7, exonuclease ExoIII, and Lambda Exo.

6. The method according to any one of the foregoing claims, **characterized in that** the template DNA is a plasmid.

7. The method according to claim 6, **characterized in that** the plasmid comprises an origin of replication and a gene of interest, and does not comprise a selection marker gene.

8. The method according to claim 7, **characterized in that** the origin of replication is selected from a pUC origin of replication, a pMB1 origin of replication and derivatives thereof, a ColEl origin of replication, and an R6Kγ origin of replication.

9. The method according to any one of the foregoing claims, **characterized in that** the one or more nicks are greater than or equal to two nicks.

10. The method according to any one of the foregoing claims, **characterized in that** a ratio of the sequence length of the template DNA to the number of nicks is less than 10 kb per nick, preferably less than 5 kb per nicking site, further preferably less than 3 kb per nicking site, further more preferably less than 2 kb per nicking site, and most preferably less than 1 kb per nicking site.

11. The method according to any one of the foregoing claims, **characterized in that** a molar ratio of the Cas nicking enzyme, the gRNA, and the template DNA is 3 : 3 : 1-12 : 12 : 1, preferably 4 : 4 : 1-11 : 11 : 1, and most preferably 5 : 5 : 1-10 : 10 : 1.

12. The method according to any one of the foregoing claims, **characterized in that** the method further comprises the following step after step 3): purifying the digestion product to obtain a high-purity circular single-stranded DNA.

13. A method for preparing a linear single-stranded DNA, **characterized in that** the method comprises the following step: cleaving the circular single-stranded (css) DNA obtained by the method according to any one of the foregoing claims to obtain a linear single-stranded (lss) DNA.

14. A kit for preparing a circular or linear single-stranded DNA, **characterized in that** the kit comprises a Cas nicking enzyme and an exonuclease.

15. The kit according to claim 14, **characterized in that** the Cas nicking enzyme is a Cas9 nicking enzyme.

16. The kit according to claim 15, **characterized in that** the Cas9 nicking enzyme is one or more selected from the group of: D10A, H840A, N863A, and N854A.

17. The kit according to any one of claims 14-16, **characterized in that** the exonuclease is one or more selected from the group of: exonuclease T7, exonuclease ExoIII, and Lambda Exo.

18. The kit according to any one of claims 14-17, **characterized in that** the kit further comprises a positive control template DNA and a positive gRNA.

19. The kit according to any one of claims 14-18, **characterized in that** the kit further comprises a template DNA and gRNA.

20. The kit according to any one of claims 14-19, **characterized in that** the kit further comprises a buffer solution.

21. The kit according to any one of claims 14-20, **characterized in that** the kit further comprises a reagent for purifying the circular or linear single-stranded DNA.

22. A circular or linear single-stranded DNA, **characterized in that** the circular or linear single-stranded DNA comprises an origin of replication and a gene of interest, and not comprising a selection marker gene.

23. The circular or linear single-stranded DNA according to claim 22, **characterized in that** the origin of replication is selected from a pUC origin of replication, a pMB1 origin of replication and derivatives thereof, a ColE1 origin of replication, and an R6Kγ origin of replication.

24. A composition, **characterized in that** the composition comprises the circular or linear single-stranded DNA according to claim 22 or 23.

25. The composition according to claim 24, **characterized in that** the composition is a pharmaceutical composition.

26. A digestion product, **characterized in that** the digestion product comprises a target strand in the form of a circular single-stranded DNA and is obtained by the method according to any one of claims 1-11.

27. The circular or linear single-stranded DNA according to claim 22 or 23, or the composition according to claim 24 or 25, or the digestion product according to claim 26 for use in gene therapy, gene recombination, DNA library construction, DNA origami, or DNA storage elements.
